(19) Europäisches Patentamt
European Patent Office
Office européen des brevets

(11) Veröffentlichungsnummer: **0 474 038 A1**

(12) # EUROPÄISCHE PATENTANMELDUNG

(21) Anmeldenummer: **91114029.1**

(22) Anmeldetag: **22.08.91**

(51) Int. Cl.5: **C07D 498/047**, A61K 31/42, C07D 205/08

(30) Priorität: **04.09.90 DE 4027928**

(43) Veröffentlichungstag der Anmeldung: **11.03.92 Patentblatt 92/11**

(84) Benannte Vertragsstaaten: **AT BE CH DE DK ES FR GB GR IT LI LU NL SE**

(71) Anmelder: **BAYER AG**
**W-5090 Leverkusen 1 Bayerwerk(DE)**

(72) Erfinder: **Wild, Hanno, Dr.**
**Ausblick 128**
**W-5600 Wuppertal 1(DE)**
Erfinder: **Schröck, Wilfried, Dr.**
**Heinrich-Heine-Strasse 45**
**W-5600 Wuppertal 11(DE)**
Erfinder: **Hartwig, Wolfgang, Dr.**
**Pahlkestrasse 3**
**W-5600 Wuppertal 1(DE)**
Erfinder: **Metzger, Karl Georg, Dr.**
**Pahlkestrasse 75**
**W-5600 Wuppertal 1(DE)**
Erfinder: **Endermann, Rainer, Dr.**
**In den Birken 152a**
**W-5600 Wuppertal 1(DE)**
Erfinder: **Bremm, Klaus Dieter, Dr.**
**Vogelsauer 59**
**W-5600 Wuppertal(DE)**

(54) **Neue 2-tert. substituierte Methyloxapenem-3-carbonsäuren und -ester, Verfahren zu ihrer Herstellung und ihre Verwendung.**

(57) Die Erfindung betrifft neue 2-tert.substituierte Methyloxapenem-3-carbonsäuren und -ester der allgemeinen Formel (I)

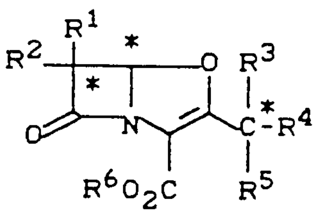

(I)

in welcher $R_1$, $R_2$, $R_3$, $R_4$, $R_5$ und $R_6$ die in der Beschreibung angegebene Bedeutung haben, Verfahren zu ihrer Herstellung und ihre Verwendung als Arzneimittel, insbesondere als antibakterielle, oral wirksame Antibiotika und $\beta$-Lactamaseinhibitoren.

EP 0 474 038 A1

Die Erfindung betrifft neue 2-tert.substituierte Methyloxapenem-3-carbonsäurenund -ester, Verfahren zu ihrer Herstellung und ihre Verwendung als Arzneimittel, insbesondere als antibakterielle, oral wirksame Antibiotika und $\beta$-Lactamaseinhibitoren.

Aus der Publikation EP 301 394 A1 und EP 362 622 A1 sind bereits stabile Oxapenem-3-carbonsäuren bekannt.

Die Erfindung betrifft neue 2-tert.substituierte Methyloxapenem-3-carbonsäurenund -ester der allgemeinen Formel (I),

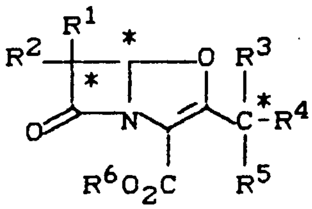

(I)

in welcher

$R^1$ und $R^2$ gleich oder verschieden sind und

- für Wasserstoff oder
- für geradkettiges oder verzweigtes Alkyl mit bis 8 Kohlenstoffatomen stehen, das gegebenenfalls durch Carboxy, Hydroxy, Halogen, Phenyl, Phenoxy, durch geradkettiges oder verzweigtes Alkoxy oder Alkoxycarbonyl mit jeweils bis zu 6 Kohlenstoffatomen oder durch eine Gruppe der Formel $-NR^7R^8$ substituiert ist,

  worin

  $R^7$ und $R^8$ gleich oder verschieden sind und Wasserstoff, Phenyl oder geradkettiges oder verzweigtes Alkyl mit bis zu 8 Kohlenstoffatomen bedeuten,

  oder

  $R^7$ und $R^8$ gemeinsam mit dem Stickstoffatom einen 5- oder 6-gliedrigen, gesättigten oder ungesättigten Heterocyclus mit bis zu 3 Heteroatomen aus der Reihe N, S oder O bilden,

  oder

  $R^7$ oder $R^8$ für eine Gruppe der Formel

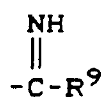 oder 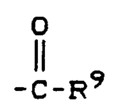

  stehen,

  worin

  $R^9$ und $R^{9'}$ gleich oder verschieden sind und

  - Wasserstoff, geradkettiges oder verzweigtes Alkyl mit bis zu 8 Kohlenstoffatomen oder Phenyl bedeuten,

$R^3$ und $R^5$ gleich oder verschieden sind und

- für geradkettiges oder verzweigtes Alkyl mit bis zu 8 Kohlenstoffatomen stehen, das gegebenenfalls durch Phenyl, Hydroxy, Halogen oder Amino substituiert ist,
- für Aryl mit 6 bis 10 Kohlenstoffatomen oder für einen 5- bis 7-gliedrigen, gesättigten oder ungesättigten Heterocyclus mit bis zu 4 Heteroatomen aus der Reihe N, O oder S stehen,

$R^4$

- für eine Gruppe der Formel $-NR^7R^8$, $-OR^{10}$ oder $-SR^{11}$ steht,

  worin

  $R^7$ und $R^8$ die oben angegebene Bedeutung haben,

  $R^{10}$

  - Wasserstoff oder geradkettiges oder verzweigtes Alkyl mit bis zu 8 Kohlenstoffatomen bedeutet, das gegebenenfalls durch Phenyl, Hydroxy, Halogen, geradkettiges oder verzweigtes Alkoxy mit bis zu 6 Kohlenstoffatomen oder durch einen gesättigten oder ungesättigten 5- bis 7-gliedrigen Heterocyclus mit bis zu 4

Heteroatomen aus der Reihe N, S oder O substituiert ist, oder

- Phenyl bedeutet, das bis zu 3-fach gleich oder verschieden durch Halogen, Trifluormethyl, Trifluormethoxy, Hydroxy oder durch geradkettiges oder verzweigtes Alkyl oder Alkoxy mit jeweils bis zu 6 Kohlenstoffatomen substituiert ist,

$R^{11}$

- Wasserstoff oder geradkettiges oder verzweigtes Alkyl mit bis zu 6 Kohlenstoffatomen bedeutet, das gegebenenfalls durch Hydroxy oder durch die Gruppe $-NR^7R^8$ substituiert ist,
  worin
  $R^7$ und $R^8$ die oben angegebene Bedeutung haben,
  oder
- Phenyl oder einen 5- bis 7-gliedrigen, gesättigten oder ungesättigten Heterocyclus mit bis zu 4 Heteroatomen aus der Reihe N, S oder O bedeutet,

$R^6$

- für Wasserstoff, oder
- für geradkettiges oder verzweigtes Alkyl mit bis zu 8 Kohlenstoffatomen steht, oder
- für einen in vivo spaltbaren Esterrest steht,

und deren physiologisch unbedenklichen Salze.

Steht $R^6$ für einen in vivo leicht abspaltbaren Esterrest, so sind hiermit pharmazeutisch verträgliche Esterreste gemeint, die in vivo leicht zu freien Carboxylgruppen ($R^6$ = H) hydrolysiert werden.

Solche Esterreste sind auf dem $\beta$-Lactam-Gebiet gut bekannt. In den meisten Fällen bessern sie die Absorptionseigenschaften der $\beta$-Lactam-Verbindungen. Außerdem sollte der Rest $R^6$ von solcher Art sein, daß er einer Verbindung der Formel (I) pharmazeutisch annehmbare Eigenschaften verleiht und bei Spaltung in vivo pharmazeutisch annehmbare Fragmente freisetzt.

Beispiele für solche Gruppen befinden sich in der DE-OS 2 517 316. Bevorzugte in vivo abspaltbare Estergruppen sind die der folgenden Formeln:

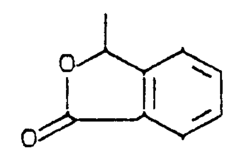 , 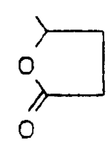 , 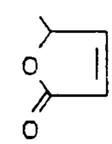 ,

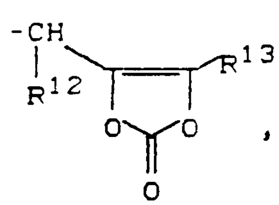 , 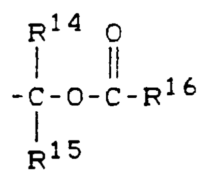 oder

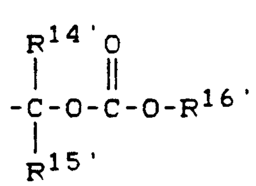

worin

$R^{12}$ und $R^{13}$ gleich oder verschieden sind und
- für Wasserstoff, Phenyl oder
- für $C_1$-$C_4$-Alkyl, bevorzugt für Methyl stehen,

$R^{14}$, $R^{14'}$, $R^{15}$ und $R^{15'}$ gleich oder verschieden sind und
- für Wasserstoff oder

- für $C_1$-$C_4$-Alkyl, bevorzugt Methyl stehen

und

$R^{16}$ und $R^{16'}$ gleich oder verschieden sind und

- für $C_1$-$C_6$-Alkyl, bevorzugt für $C_1$-$C_4$-Alkyl stehen,

Die Verbindungen der Formel (I) können als freie Säuren, Ester, als innere Salze oder als nicht-toxische pharmazeutisch verträgliche Salze der sauren Carboxylgruppen, wie Natrium-, Kalium-, Magnesium-, Calcium-, Aluminium- oder Ammoniumsalze, mit Aminen wie Di-, Tri-niedrigalkylaminen, Procain, Dibenzylamin, N,N'-Dibenzylethylendiamin, N-Benzyl-$\beta$-phenyl-ethylamin, N-Methyl und N-Ethylmorpholin, 1-Ephenamin, Dihydroabiethylamin, N,N'-Bis-dehydroabiethylethylendiamin, N-Niedrigalkylpiperidin und anderen Aminen, die zur Bildung von Salzen von Penicillinen und Cephalosporinen verwendet werden können, vorliegen.

Als nicht-toxische, pharmazeutisch verträgliche Salze der basischen Aminogruppen mit anorganischen oder organischen Säureresten sind beispielsweise Chlorid, Bromid, Iodid, Sulfat, Hydrogensulfat, Phosphat, Hydrogenphosphat, Carbonat, Hydrogencarbonat, oder Sulfonate wie Methylsulfonat, Ethansulfonat, Toluolsulfonat, Benzolsulfonat, Naphthalindisulfonat, oder Carboxylate wie Acetat, Formiat, Oxalat, Tartrat, Citrat, Maleat, Fumarat, Benzoat, Succinat oder Lactat bevorzugt zu nennen.

Die erfindungsgemäßen Verbindungen der allgemeinen Formel (I) besitzen mindestens 1 bis 3 asymmetrische Kohlenstoffatome (*), die unabhängig voneinander in der R-oder S- Konfiguration vorliegen können.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher

$R^1$ und $R^2$ gleich oder verschieden sind und

- für Wasserstoff oder
- für geradkettiges oder verzweigtes Alkyl mit bis zu 6 Kohlenstoffatomen stehen, das gegebenenfalls durch Carboxy, Hydroxy, Phenyl, Phenoxy oder durch eine Gruppe der Formel $-NR^7R^8$ substituiert ist,

  worin

  $R^7$ und $R^8$ gleich oder verschieden sind und

  - Wasserstoff, Phenyl oder geradkettiges oder verzweigtes Alkyl mit bis zu 6 Kohlenstoffatomen bedeuten,

  oder

  $R^7$ und $R^8$ gemeinsam mit dem Stickstoffatom einen Morpholino-, Tetrazolyl- oder Triazolylring bilden,

  oder

  $R^7$ oder $R^8$ für eine Gruppe der Formel

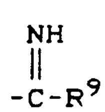

oder

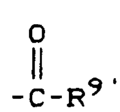

  stehen

  worin

  $R^9$ und $R^{9'}$ gleich oder verschieden sind und

  - Wasserstoff, geradkettiges oder verzweigtes Alkyl mit bis zu 6 Kohlenstoffatomen oder Phenyl bedeuten,

$R^3$ und $R^5$ gleich oder verschieden sind und

- für geradkettiges oder verzweigtes Alkyl mit bis zu 6 Kohlenstoffatomen stehen, das gegebenenfalls durch Phenyl, Hydroxy, Fluor oder Chlor substituiert ist,
- für Phenyl, Triazolyl, Pyridyl, Tetrazolyl, Thienyl, Oxazolyl, Isoxazolyl oder Thiazolyl stehen,

$R^4$

- für eine Gruppe der Formel $-NR^7R^8$, $-OR^{10}$ oder $-SR^{11}$ steht,
  worin
  $R^7$ und $R^8$ die oben angegebene Bedeutung haben,
  $R^{10}$
  - Wasserstoff oder geradkettiges oder verzweigtes Alkyl mit bis zu 6 Kohlenstoffatomen bedeutet, das gegebenenfalls durch Phenyl, Hydroxy, Tetrazolyl oder Triazolyl substituiert ist,
  - Phenyl bedeutet, das gegebenenfalls bis zu 2-fach gleich oder verschieden durch Fluor, Chlor, Brom, Hydroxy oder durch geradkettiges oder verzweigtes Alkyl mit bis zu 4 Kohlenstoffatomen substituiert ist,

  $R^{11}$
  - Wasserstoff oder geradkettiges oder verzweigtes Alkyl mit bis zu 4 Kohlenstoffatomen bedeutet, das gegebenenfalls durch die Gruppe der Formel $-NR^7R^8$ substituiert ist,
    worin
    $R^7$ und $R^8$ die oben angegebene Bedeutung haben,
  - Phenyl, Tetrazolyl, Triazolyl, Pyridyl, Thiazolyl, Oxazolyl oder Isoxazolyl bedeutet,

$R^6$

- für Wasserstoff, oder
- für geradkettiges oder verzweigtes Alkyl mit bis zu 6 Kohlenstoffatomen steht
- für einen Rest der Formel

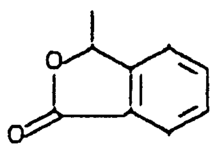 , 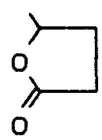 , 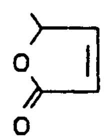 ,

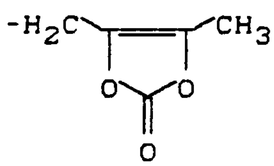 ,

$-CH_2-OCO-C(CH_3)_3$,
$-CH(CH_3)-OCOOC_2H_5$ oder
$-CH_2-OCOCH_3$ steht

und deren physiologisch unbedenklichen Salze.

Besonders bevorzugt sind Verbindungen der allgemeinen Formel (I), in welcher

$R^1$ und $R^2$ gleich oder verschieden sind und

- für Wasserstoff, oder
- für geradkettiges oder verzweigtes Alkyl mit bis zu 4 Kohlenstoffatomen stehen, das gegebenenfalls durch Carboxy, Hydroxy oder durch eine Gruppe $-NR^7R^8$ substituiert ist,
  worin
  $R^7$ und $R^8$ gleich oder verschieden sind und
  - Wasserstoff oder geradkettiges oder verzweigtes Alkyl mit bis zu 4 Kohlenstoffatomen bedeuten,

$R^3$ und $R^5$ gleich oder verschieden sind und

- für geradkettiges oder verzweigtes Alkyl mit bis zu 4 Kohlenstoffatomen stehen, das

gegebenenfalls durch Phenyl, Hydroxy oder Chlor substituiert ist,
- für Phenyl, Triazolyl, Tetrazolyl, Thienyl oder Thiazolyl stehen,

$R^4$
- für eine Gruppe der Formel $-NR^7R^8$, $-OR^{10}$ oder $-SR^{11}$ steht,
worin
$R^7$ und $R^8$ die oben angegebene Bedeutung haben,
$R^{10}$
- Wasserstoff oder geradkettiges oder verzweigtes Alkyl mit bis zu 4 Kohlenstoffatomen bedeutet, das gegebenenfalls durch Hydroxy substituiert ist,
- Phenyl bedeutet, das gegebenenfalls durch Fluor, Chlor oder Hydroxy substituiert ist,

$R^{11}$ - Methyl, Ethyl, Phenyl, Tetrazolyl, Triazolyl oder Thiazolyl bedeutet,

$R^6$
- für Wasserstoff oder
- für geradkettiges oder verzweigtes Alkyl mit bis zu 4 Kohlenstoffatomen steht

und deren physiologisch unbedenklichen Salze.

Die erfindungsgemäßen Verbindungen der allgemeinen Formel (I), können hergestellt werden, indem man Verbindungen der allgemeinen Formel (II)

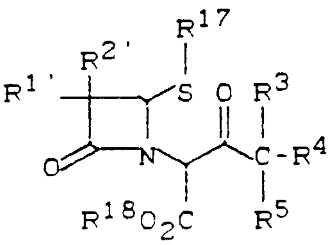

(II)

in welcher
$R^{1'}$ und $R^{2'}$ die oben angegebene Bedeutung von $R^1$ und $R^2$ haben oder für einen dort definierten Rest stehen, der durch eine Schutz- bzw. Markierungsgruppe substituiert ist,

$R^{17}$ - für geradkettiges oder verzweigtes Alkyl mit bis zu 6 Kohlenstoffatomen oder Phenyl steht, und

$R^{18}$ - für eine Carboxyschutzgruppe steht,

zunächst in inerten Lösemitteln mit Halogenierungsmitteln zu Verbindungen der allgemeinen Formel (III)

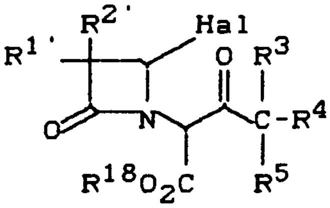

(III)

in welcher
$R^{1'}$, $R^{2'}$, $R^3$, $R^4$, $R^5$ und $R^{18}$ die oben angegebene Bedeutung haben,
und
Hal für Halogen, vorzugsweise für Chlor steht,
umsetzt, in einem nächsten Schritt unter Einwirkung von Basen in inerten Lösemitteln cyclisiert, und im Fall, daß $R^{1'}$ oder $R^{2'}$ für einen durch eine Schutz- oder Markierungsgruppe substituierten Rest steht, diese nach üblicher Methode, beispielsweise durch Hydrierung abspaltet.

Das erfindungsgemäße Verfahren kann durch folgendes Formelschema beispielhaft erläutert werden:

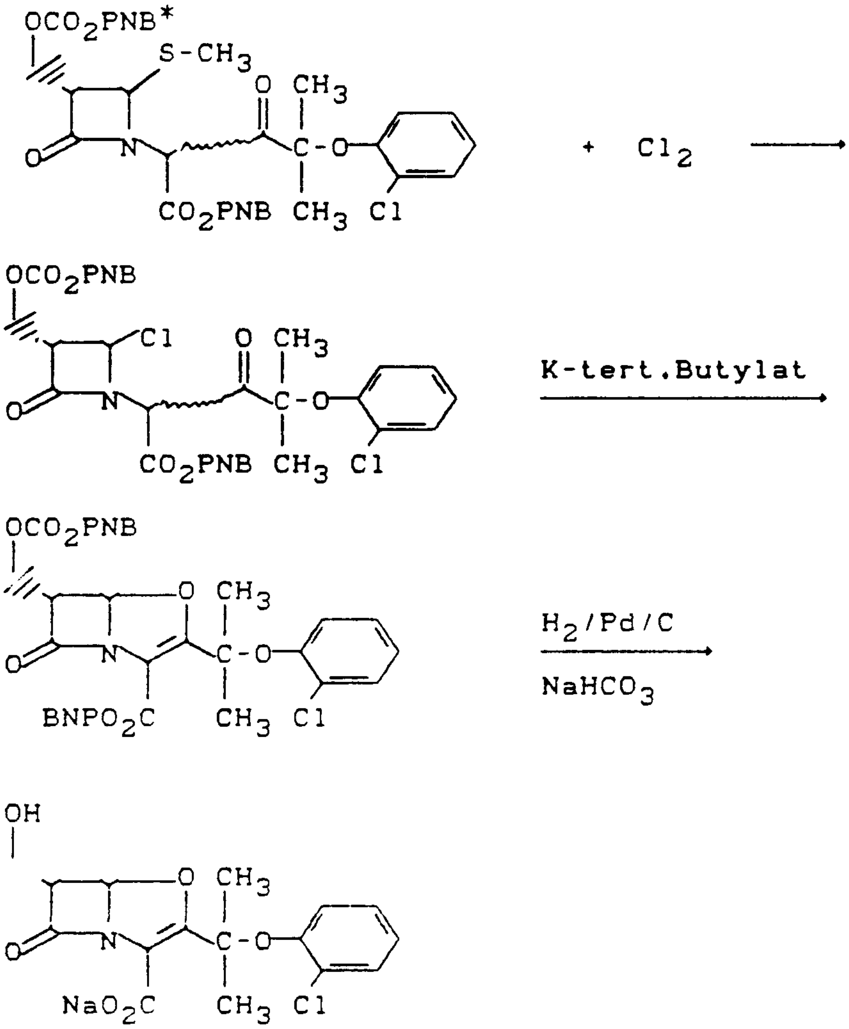


* PNB =
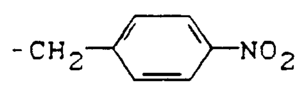

Als Schutz- bwz. Markierungsgruppe im Rahmen der oben angegebenen Definitionen für $R^{1'}$, $R^{2'}$ und $R^{18}$ stehen im allgemeinen die in der $\beta$-Lactam-Chemie üblichen Carboxyschutzgruppen. Bevorzugt sind leicht abspaltbare Gruppen wie beispielsweise; tert.Butyl, 2,2,2-Trichlorethyl, Diphenylmethyl, Triphenylmethyl, Acetoxymethyl, Allyl, Benzyl, 4-Methoxyphenyl, 4-Nitrobenzyl, 2-Nitrobenzyl, 4-Methoxybenzyl, 2,4-Dimethoxybenzyl, Trimethylsilylethyl, Trimethylsilyl, tert.Butyl-dimethylsilyl, Acetonyl, 1-Phenoxyethyl oder 2-Methyl-2-propenyl oder substituierte oder unsubstituiertes $C_1$-$C_6$-Alkyl, Aryl-($C_1$-$C_6$)-alkyl oder $C_1$-$C_6$-Alkenyl, wie Trimethoxybenzyl, 2-Oxapropyl, 2-Oxo-2-phenylethyl, Allyl, 2-Cyanoethyl, 2-Trimethylsilyloxyethyl, 2,2,2-Trichlorethyl, Pivaloyloxymethyl oder Brom-tert.butyl zu nennen.

Als Lösemittel für die Halogenierung eignen sich die üblichen organischen Lösemittel, die sich unter den Reaktionsbedingungen nicht verändern. Hierzu gehören bevorzugt Ether wie Diethylether, Butylmethylether, Dioxan oder Tetrahydrofuran, oder Kohlenwasserstoffe wie Benzol, Toluol, Xylol, oder Cyclohexan, oder Amide wie Dimethylformamid oder Hexamethylphosphorsäuretriamid, oder Alkohole wie Methanol, Ethanol, Propanol oder Isopropanol, oder Chlorkohlenwasserstoffe wie Methylenchlorid, Chloroform oder Tetrachlorkohlenstoff, oder Aceton, Acetonitril oder Essigester. Bevorzugt sind Tetrachlorkohlenstoff, Toluol oder Methylenchlorid.

Die Halogenierung wird in einem Temperaturbereich von -100 °C bis +30 °C, vorzugsweise bei -70 °C bis 0 °C durchgeführt.

Als Halogenierungsmittel eigenen sich beispielsweise Chlor, Brom, Jod oder Sulfurylchlorid. Bevorzugt ist Chlor.

Die Halogenierungsmittel werden in einer Menge von 1 bis 20 mol, bevorzugt von 1 bis 3 Mol, bezogen auf 1 Mol der Verbindungen der allgemeinen Formel (II) eingesetzt.

Für die Cyclisierung eignen sich als Lösemittel Ether, wie Diethylether, Butylmethylether, Dioxan oder Tetrahydrofuran, oder Kohlenwasserstoffe wie Benzol, Toluol, Xylol, oder Cyclohexan, oder Dimethylformamid. Bevorzugt sind Tetrahydrofuran, Dioxan und Dimethylformamid.

Als Basen eignen sich für die Cyclisierung Alkoholate wie Natriummethanolat, Kalium-tert.butylat, Natriumphenolat, Natriumthiophenolat oder Diazabicycloundecen oder Triethylamin. Bevorzugt sind Kalium-tert.butylat oder Triethylamin.

Die Base wird in einer Menge von 1 bis 3, bevorzugt von 1 bis 1,5 Mol, bezogen auf 1 Mol der Verbindungen der allgemeinen Formel (III) eingesetzt.

Die Abspaltung der Schutz- oder Maskierungsgruppen ($R^{1'}$, $R^{2'}$, $R^{18}$) erfolgt nach bekannten Methoden, wie beispielsweise durch katalytische Hydrierung, Hydrolyse, Reduktion, nucleophile Substitution oder Solvolyse. Bevorzugt ist die katalytische Hydrierung.

Geeignete Hydrierungskatalysatoren sind beispielsweise Platiniummetalle und deren Oxide, Raney-Nickel oder Palladium auf Kohle. Bevorzugt ist der Einsatz von Palladium auf Kohle.

In Anwesenheit von Wasserstoff eignen sich als Lösemittel für die Hydrierung Alkohole wie Methanol, Ethanol, Essigester oder Gemische wie Essigester/$H_2O$ oder Ethanol/$H_2O$. Gegebenenfalls wird die Hydrierung in Anwesenheit einer schwachen Base, beispielsweise Natriumhydrogencarbonat durchgeführt.

Die Hydrierung wird in einem Temperaturbereich von 0 bis 30 °C, vorzugsweise von 0 bis 10 °C durchgeführt.

Die hydrolytische Abspaltung der Schutzgruppe erfolgt nach üblichen Methoden in einem der oben aufgeführten Lösemitteln, vorzugsweise in Tetrahydrofuran oder Tetrahydrofuran/$H_2O$ in Anwesenheit einer Base, beispielsweise wäßriger verdünnter Natronlauge in einem Temperaturbereich von -50 °C bis +40 °C, vorzugsweise bei -40 °C bis +25 °C.

In einem Temperaturbereich von -50 °C bis +40 °C, vorzugsweise von -30 °C bis +25 °C werden der reduktive, nucleophile und die solvolytische Abspaltung der Schutzgruppen durchgeführt.

Als Reduktionsmittel bei reduktiver Abspaltung eignen sich die üblichen Reduktionsmittel wie beispielsweise Zinkstaub. Bei der nucleophilen Substitution werden nach üblicher Methode nucleophile Agentien wie beispielsweise Tetrabutylammoniumfluorid eingesetzt. Die Solvolyse erfolgt zunächst unter Einsatz einer Lewis-Säure, wie beispielsweise Aluminiumtrichlorid und anschließender Zugabe eines solvolysierenden Lösemittels, beispielsweise Wasser.

Als Lösemittel eignen sich die oben aufgeführten Lösemittel. Bevorzugt ist für die reduktive Abspaltung ein Essigsäure/Wasser-Gemisch, für die nucleophile Substitution und die Solvolyse Tetrahydrofuran.

Im allgemeinen werden die Umsetzungen bei Normaldruck durchgeführt. Es ist aber auch möglich, bei Unterdruck oder bei Überdruck zu arbeiten (z.B. von 0,5 bis 5 bar).

Die Verbindungen der allgemeinen Formel (III) sind neu und können nach dem oben aufgeführten Verfahren hergestellt werden.

Die Verbindungen der allgemeinen Formel (II) sind ebenfalls neu und können hergestellt werden, indem man

Verbindungen der allgemeinen Formel (IV)

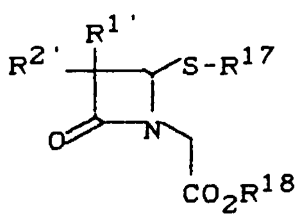

(IV)

in welcher

$R^{1'}$, $R^{2'}$, $R^{17}$ und $R^{18}$ die oben angegebene Bedeutung haben,

mit Säurehalogeniden der allgemeinen Formel (V)

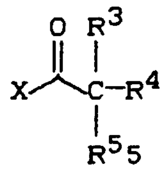 (V)

in welcher
$R^3$, $R^4$ und $R^5$ die oben angegebene Bedeutung haben,
und
X - für Halogen, vorzugsweise für Chlor steht,
in inerten Lösemitteln in Anwesenheit einer Base umsetzt.

Als Lösemittel eignen sich die oben aufgeführten Lösemittel. Bevorzugt wird Tetrahydrofuran eingesetzt.

Als Basen eignen sich Lithiumamide oder -alkyle wie beispielsweise Butyllithium, Lithiumdiisopropylamid oder Lithium-bis-(trimethylsilylamid). Bevorzugt ist Lithium-bis-(trimethylsilylamid).

Die Base wird in einer Menge von 1 bis 5, bevorzugt von 2 bis 3 Mol eingesetzt.

Im allgemeinen wird die Umsetzung bei Normaldruck durchgeführt. Es ist aber auch möglich, bei Unterdruck oder bei Überdruck zu arbeiten (z.B. von 0,5 bis 5 bar).

Gegebenenfalls können die Säurehalogenide der allgemeinen Formel (V) auch in situ hergestellt werden, indem man zunächst ausgehend von der entsprechenden Säure nach üblichen Methoden mit einem typischen Halogenierungsmittel, in diesen Fällen bevorzugt mit Oxalylchlorid umsetzt.

Die Verbindungen der allgemeinen Formel (IV) sind bekannt oder können nach bekannten Methoden hergestellt werden [vgl. EP 301 394 A1].

Die Verbindungen der allgemeinen Formel (V) sind an sich bekannt oder können ebenfalls nach literaturbekannten Verfahren hergestellt werden [vgl. Synthesis 1982, 521].

Die erfindungsgemäßen Verbindungen der allgemeinen Formel (I) weisen bei geringer Toxizität ein breites antibakterielles Spektrum gegen gram-positive und gramnegative Keime auf. Diese Eigenschaften ermöglichen ihre Verwendung als chemotherapeutische Wirkstoffe in der Human- und Tiermedizin.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch solche Erreger hervorgerufen werden.

Die erfindungsgemäßen Verbindungen weisen außerdem eine hohe Affinität gegen $\beta$-Lactamasen grampositiver, insbesondere Staphylokokken, und gram-negativer Keime auf. Sie sind daher auch gegen $\beta$-Lactamase bildende Bakterien geeignet.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls mit einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

Die neuen Verbindungen können in den üblichen Konzentrationen und Zubereitungen zusammen mit

dem Futter bzw. Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit Aminoglykosidantibiotica, wie z.B. Gentamicin, Sisomicin, Kanamicin, Amikacin oder Tobramicin kombiniert werden.

## Ausgangsverbindungen

### Beispiel 1

4-Methylthioazetidin-2-on

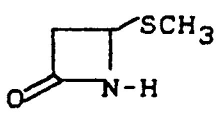

450,8 g 4-Benzoyloxyazetidin-2-on werden in 1,7 l Acetonitril gelöst. Unter Eiskühlung tropft man eine Lösung von 182 g Natriummethanthiolat in 1,7 l Wasser zu und rührt 30 min bei 20 °C nach. Zur Aufarbeitung wird mit Dichlormethan verdünnt, die wäßrige Phase abgetrennt und mit Dichlormethan zweimal extrahiert. Die organischen Phasen werden vereinigt, gewaschen (1 x ges. $NaHCO_3$-Lösung, 1 x ges. NaCl-Lösung), getrocknet ($MgSO_4$) und eingedampft. Kristallisation aus Ether bei -78 °C liefert 237 g Produkt mit Fp. 57-59 °C.

### Beispiel 2

1-(Dimethylethyldimethylsilyl)-4-methylthioazetidin-2-on

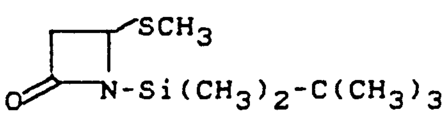

138,5 g der Verbindung aus Beispiel 1 und 204,5 g Dimethylethyldimethylsilylchlorid werden in 760 ml Dimethylformamid bei 0 °C portionsweise mit 165,5 g Diazabicyclooctan versetzt. Man rührt 1 h bei 0 °C, 2 h bei 20 °C nach, versetzt mit Essigester und Wasser, trennt die organische Phase ab und wäscht sie zweimal mit Wasser. Nach Trocknen ($MgSO_4$) und Einengen wird der Rückstand bei 0,05 mbar destilliert. Man erhält 261 g des Produkts als farblose Flüssigkeit mit Sdp. 80-86 °C.

### Beispiel 3

(trans)-1-(Dimethylethyldimethylsilyl)-3-hydroxymethyl-4-methylthioazetidin-2-on

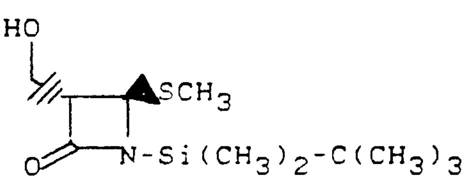

Zu einer Lösung von 0,55 mol Lithiumdiisopropylamid in 1 l Tetrahydrofuran tropft man bei -65 °C 116 g der Verbindung aus Beispiel 2 in 500 ml Tetrahydrofuran innerhalb von 15 min zu und rührt weitere 15 min nach. Dann wird über 3 h in die auf -78 °C gekühlte Reaktionslösung ein Strom von Formaldehyd eingeleitet. Anschließend wird die kalte Lösung in 5 l Wasser eingetragen und das Gemisch mit Dichlormethan erschöpfend extrahiert.

Die organischen Phasen werden getrocknet ($MgSO_4$), eingedampft und der Rückstand an Kieselgel gereinigt (Toluol/Essigester 3:1). Man erhält 50 g an Produkt als gelbes Öl, das bei -10 ° C aus Petrolether kristallisiert (Fp. 43 ° C).

Beispiel 4

(trans)-1-(Dimethylethyldimethylsilyl)-4-methylthio-3-p-nitrobenzyloxycarbonyloxymethylazetidin-2-on

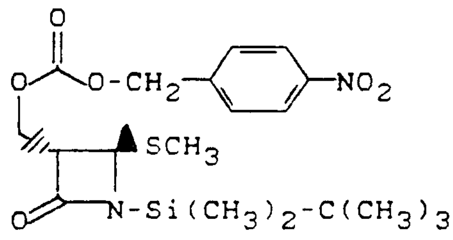

77,8 g der Verbindung aus Beispiel 3 und 85,2 g Chlorameisensäure-p-nitrobenzylester in 1,5 l Dichlormethan werden bei -15 ° C portionsweise mit 48,3 g 4-Dimethylaminopyridin versetzt. Die entstandene Suspension wird 3 h bei 20 ° c nachgerührt, mit Dichlormethan verdünnt und gewaschen (1 x 0,5 N Salzsäure, 1 x ges. NaCl-Lösung). Nach Trocknen ($MgSO_4$) und Einengen erhält man einen Rückstand, der an Kieselgel gereinigt wird (Toluol/Essigester 20:1). Es werden 124 g Carbonat als farbloses Öl erhalten.

$^1$H-HMR ($CDCl_3$): $\delta$ = 0,28 (s, 3H); 0,034 (s, 3H); 0,98 (s, 9H); 2,10 (s, 3H); 3,52 (m, 1H); 4,42 (dd, 1H); 4,52 (dd, 1H); 4,60 (d, 1H); 5,26 (s, 2H); 7,52 (d, 2H); 8,23 (d, 2H).

Beispiel 5

(trans)-4-Methylthio-3-p-nitrobenzyloxycarbonyloxymethylazetidin-2-on

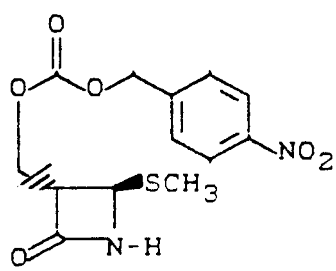

77 g der Verbindung aus Beispiel 4 in 875 ml Tetrahydrofuran werden mit 21,7 ml Eisessig versetzt und dann werden bei 0 ° C 209 ml 1 M Tetrabutylammoniumfluorid-Lösung in Tetrahydrofuran zutropft. Nach 15 min bei 0 ° C versetzt man mit Dichlormethan, wäscht zweimal mit ges. NaCl-Lösung, trocknet ($MgSO_4$) und engt ein. Der Rückstand wird an Kieselgel gereinigt (Toluol/Essigester 2:1) und liefert 52 g des Produktes als farbloses Öl.

$^1$H-NMR ($CDCl_3$): $\delta$ = 2,15 (s, 3H); 3,49 (m, 1H); 4,49 (dd, 1H); 4,55 (dd, 1H); 4,73 (d, J = 2 Hz, 1H); 5,28 (s, 2H); 6,37 (breit, 1H); 7,51 (d, 2H); 8,21 (d, 2H).

Beispiel 6

(trans)-[4-Methylthio-3-p-nitrobenzyloxycarbonyloxymethyl-2-oxo-azetidinyl]-essigsäure-p-nitrobenzylester

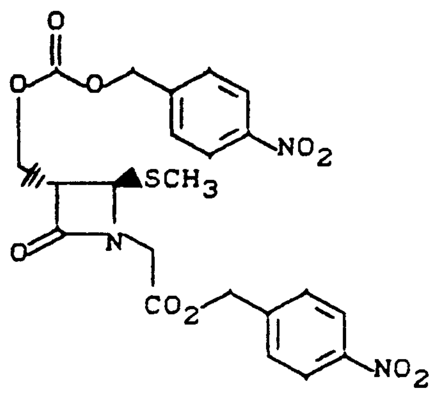

48,9 g der Verbindung aus Beispiel 5 und 205,5 g Bromessigsäure-p-nitrobenzylester werden in 1,5 l Tetrahydrofuran und 225 ml Dimethylformamid bei -35 ° C mit 165 ml einer 1 M Lithiumbistrimethylsilylamid-Lösung in Tetrahydrofuran während 15 min versetzt. Nach weiteren 15 min bei -35 ° C werden 150 ml ges. $NH_4Cl$-Lösung eingetragen und anschließend zwischen Essigester und 1 N Salzsäure verteilt, Die organische Phase wird getrocknet ($MgSO_4$), eingeengt und der Rückstand an Kieselgel zweimal gereinigt (1. Petrolether/Ether 1:1, 2. Dichlormethan/Aceton 50.1). Es werden 44 g des Esters als hellgelbes Öl erhalten,

$^1$H-NMR ($CDCl_3$): $\delta$ = 2,03 (s, 3H); 3,58 (m, 1H); 3,83 (d, 1H); 4,32 (d, 1H); 4,54 (d, 2H); 5,26 (s, 4H); 7,52 (d, 2H); 7,55 (d, 2H); 8,23 (d, 4H).

Beispiel 7

4-Methylthio-3-(p-nitrobenzyloxycarbonyloxymethyl)-1-[1-p-nitrobenzyloxycarbonyl-1-(1-p-chlorphenyloxy-1,1-dimethyl)methylcarbonyl]methyl-azetidin-2-on

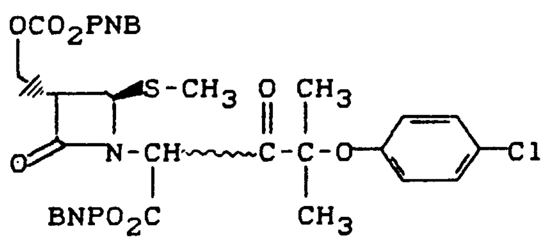

1,98 g (9,24 mmol) 2-p-Chlorphenoxy-isobuttersäure werden in 18 ml abs. THF gelöst, mit 1,21 ml Oxalylchlorid versetzt und 2 h bei 60 ° C gerührt. Man zieht das Solvens im Vakuum ab und löst den Rückstand in 10 ml abs. THF (Lösung 1). Zur Lösung von 4 g (7,7 mmol) 4-Methylthio-3-(p-nitrobenzyloxycarbonyloxy)methyl-1-(p-nitrobenzyloxycarbonyl)methyl-azetidin-2-on gibt man bei -70 ° C nacheinander Lösung 1 und 16,6 ml einer 1-M-Lösung von Lithium-bis-trimethylsilylamid so zu, daß die Temperatur -60 ° C nicht übersteigt . Man rührt 1,5 h bei -70 ° C, gibt 200 ml Essigester zu und wäscht die organische Phase je mit 150 ml 1 N HCl, 150 ml Wasser und 200 ml gesättigter Kochsalzlösung. Man trocknet die organische Phase über $MgSO_4$, zieht das Solvens im Vakuum ab und chromatographiert den Rückstand über Kieselgel mit Eluens Toluol/ Essigester = 5:1. Man erhält 2,07 g (37,5% der Theorie) der Titelverbindung mit $R_f$ = 0,45 (Toluol/Essigester 3:1).

Beispiel 8

4-Methylthio-3-(p-nitrobenzyloxycarbonyloxymethyl)-1-[1-p-nitrobenzyloxycarbonyl-1-(1-o-chlorphenyloxy-1,1-dimethyl)methylcarbonyl]methyl-azetidin-2-on

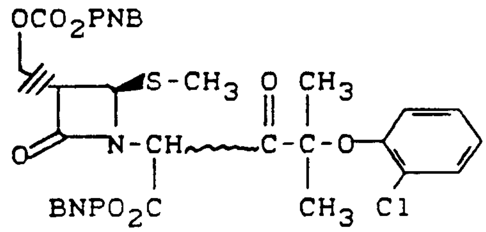

Man verfährt analog Beispiel 7 und erhielt ausgehend von 2,48 g (11,5 mmol) 1-o-Chlorphenyl-isobuttersäure 2,4 g (34,8% der Theorie) der Titelverbindung mit $R_f$ = 0,41 (Toluol/Essigester = 3/1).

Beispiel 9

4-Chlor-3-(p-nitrobenzyloxycarbonyloxymethyl)-1-[1-p-nitrobenzyloxycarbonyl-1-(1-p-chlorphenyloxy-1,1-dimethyl)methylcarbonyl]-methyl-azetidin-2-on

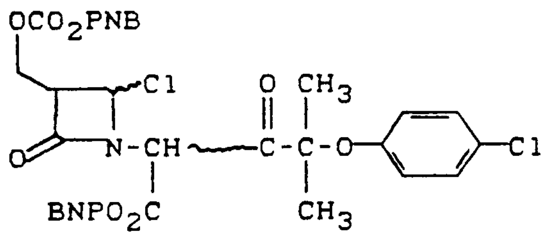

2,07 g (2,89 mmol) der Verbindung aus Beispiel 7 werden in 115 ml abs, Dichlormethan gelöst, auf -40 ° c gekühlt und langsam mit 3,6 ml einer 1,46 molaren Lösung von Chlor in Tetrachlorkohlenstoff versetzt, Man rührt 10 min bei -40 ° C, zieht das Solvens im Vakuum ab (Badtemperatur 20 ° C), nimmt den Rückstand in 100 ml Dichlormethan auf und zieht das Solvens im Vakuum ab. Der Rückstand (1,9 g, 93% der Theorie) wird 1 h am Hochvakuum getrocknet und direkt weiter umgesetzt.

Beispiel 10

4-Chlor-3-(p-nitrobenzyloxycarbonyloxymethyl)-1-[1-p-nitrobenzyloxycarbonyl-1-(1-o-chlorphenyloxy-1,1-dimethyl)methylcarbonyl]methyl-azetidin-2-on

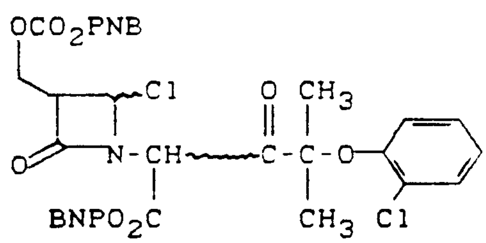

Man verfährt analog Beispiel 9. Eingesetzt werden 2,4 g (3,3 mmol) der Verbindung aus Beispiel 8. Man erhält 2,25 g (95% der Theorie) der Titelverbindung mit $R_f$ = 0,52 ($CH_2Cl_2$/Aceton = 10/1), die direkt weiterverarbeitet werden.

Beispiel 11

3-(1,1-Dimethyl-1-p-chlorphenoxy)methyl-6-(p-nitrobenzyloxycarbonyloxymethyl)-7-oxo-4-oxa-1-azabicyclo-[3.2.0]hept-2-en-2-carbonsäure-p-nitrobenzylester

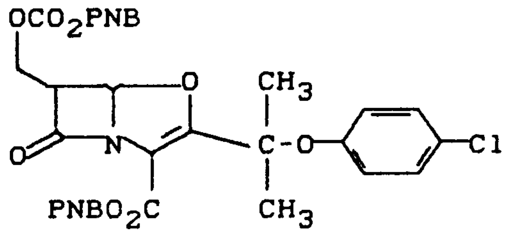

1,1 g der Verbindung aus Beispiel 7 werden in 31 ml abs. Tetrahydrofuran gelöst, auf -70°C gekühlt und mit 1,82 ml einer 0,9 M Lösung von K-tert.butylat in tert.Butanol versetzt. Man läßt 40 min bei -20°C rühren, verdünnt mit 150 ml Essigester, wäscht mit 100 ml gesättigter Kochsalzlosung, trocknet über $MgSO_4$ und zieht das Solvens im Vakuum ab. Der Rückstand wird über Kieselgel 60 (Merck) chromatographiert mit Toluol/Essigester 95:5 als Eluens. Man erhält 440 mg (24,4% der Theorie) der Titelverbindung als cis-/trans-Gemisch (cis/trans 1/6) mit $R_f$ = 0,57 und 0,50 (Toluol/Essigester 1:1).

Beispiel 12

3-(1,1-Dimethyl-1-o-chlorphenoxy)methyl-6-(p-nitrobenzyloxycarbonyloxymethyl)-7-oxo-4-oxa-1-azabicyclo-[3.2.0]hept-2-en-2-carbonsäure-p-nitrobenzylester

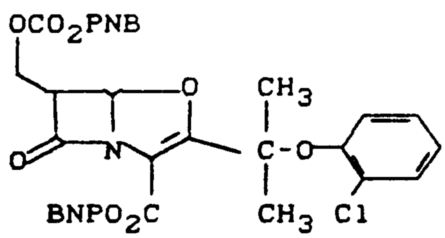

Man verfährt analog Beispiel 11. Eingesetzt werden 2,28 g (3,2 mmol) der Verbindung aus Beispiel 8. Man erhält 530 mg (24,8% der Theorie) der Titelverbindung.

$^1$H-NMR ($CDCl_3$, 250 MHz): 5,6 cis $\delta$ = 1,56 und 1,72 (je s, $C(CH_3)_2$, 6H); 4,48 (M-Teil von ABMX-Signal, $J_{AM} = J_{BM} = 5{,}6$ Hz, $J_{MX}$ = 0,7 Hz, C(6)-H, 1H); 5,23 und 5,49 (AB-Signal, $J_{AB}$ = 12,5 Hz, C-$OCH_2$Ar, 2H); 5,27 (s, $CO_2CH_2$Ar, 2H); 6,01 (d, J = 0,7 Hz, C(5)-H, 1H).

trans: $\delta$ = 1,75 und 1,78 (je s, $C(CH_3)_2$, 6H); 4,05 (t, J = 5,6 Hz, C(6)-H, 1H); 5,23 und 5,40 (AB-Signal, $J_{AB}$ = 12,5 Hz, $COCH_2$Ar, 2H); 5,96 (s, C-(5)-H, 1H).

cis/trans: $\delta$ = 4,55 (m, C(5)-$CH_2$, 2H); 6,9, 7,2, 7,55 und 8,3 (je m, ArH, 14H).

Herstellungsbeispiele (allgemeine Formel (I))

Beispiel I

[5,6 cis/trans]-3-(1,1-Dimethyl-1-p-chlorphenoxy)methyl-6-hydroxymethyl-7-oxo-4-oxa-1-azabicyclo[3.2.0]-hept-2-en-2-carbonsäure-NaSalz

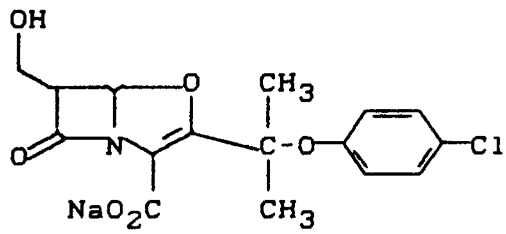

440 mg (0,66 mmol) der Verbindung aus Beispiel 11 werden in 7 ml Essigester gelöst und zu 0,5 g bei 0 ° C vorhydriertem Katalysator Pd/C zugegeben und bei 0 ° C unter Normaldruck hydriert. Nach Beendigung der Wasserstoffaufnahme wird über Kieselguhr abgesaugt, mit wenig Essigester nachgewaschen, 100 ml bidest. Wasser zugegeben und unter kräftigem Rühren bei 0 ° C mit 0,2 M NaOH pH 7,9 eingestellt. Die wässrige Phase wird abgetrennt, mit Essigester gewaschen und lyophilisiert. Man erhält 120 mg (48,5% der Theorie) (cis/trans: 1/6) der Titelverbindung als farbloses Lyophilisat.

$^1$H-NMR ($D_2O$, 300 MHz): trans: $\delta$ = 5,77 (s, C(5)-H, 1H); 6,97 und 7,28 (je m, ArH, 4H).
cis: $\delta$ = 5,83 (d, J = 0,7 Hz, C(5)-H, 1H); 6,82 und 7,22 (je m, ArH, 4H).
cis/trans: $\delta$ = 1,51 und 1,67 (je s, $C(CH_3)_2$, 6H); 3,85 (m, C(6)-$CH_2$ und C-(6)-H, 3H).

Beispiel II

[5,6-cis/trans]-3-(1,1-Dimethyl-1-o-chlorphenoxy)methyl-6-hydroxymethyl-7-oxo-4-oxa-1-azabicyclo[3.2.0]-hept-2-en-2-carbonsäure-Na-Salz

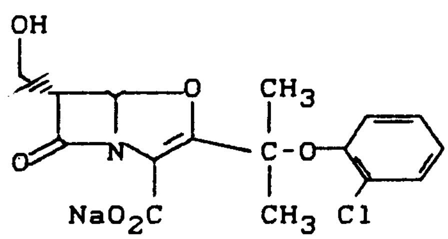

Man verfährt analog Beispiel I. Eingesetzt werden 530 mg (0,79 mmol) der Verbindung aus Beispiel 12. Man erhält 160 mg (53,7% der Theorie) der Titelverbindung als farbloses Lyophilisat.

$^1$H-NMR ($D_2O$, 300 MHz): cis: $\delta$ = 5,94 (d, J = 0,7 Hz, C(5)-H, 1H).
trans: $\delta$ = 5,15 (s, C(5)-H), 1H).
cis/trans: $\delta$ = 1,71 und 1,76 (je s, $C(CH_3)_2$, 6H); 3,87 (m, C(6)-$CH_2$ und C-(6)-H, 3H); 7,20 (m, ArH, 4H).

## Patentansprüche

1. 2-tert.substituierte Methyloxapenem-3-carbonsäuren der allgemeinen Formel (I)

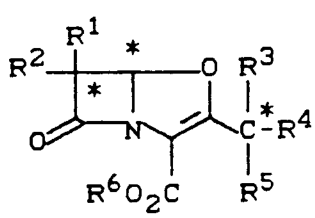

(I)

in welcher

$R^1$ und $R^2$ gleich oder verschieden sind und

- für Wasserstoff oder
- für geradkettiges oder verzweigtes Alkyl mit bis 8 Kohlenstoffatomen stehen, das gegebenenfalls durch Carboxy, Hydroxy, Halogen, Phenyl, Phenoxy, durch geradkettiges oder verzweigtes Alkoxy oder Alkoxycarbonyl mit jeweils bis zu 6 Kohlenstoffatomen oder durch eine Gruppe der Formel $-NR^7R^8$ substituiert ist,

worin

$R^7$ und $R^8$ gleich oder verschieden sind und Wasserstoff, Phenyl oder geradkettiges oder verzweigtes Alkyl mit bis zu 8 Kohlenstoffatomen bedeuten,

oder

$R^7$ und $R^8$ gemeinsam mit dem Stickstoffatom einen 5- oder 6-gliedrigen, gesättigten oder ungesättigten Heterocyclus mit bis zu 3 Heteroatomen aus der Reihe N, S oder O bilden,

oder

$R^7$ oder $R^8$ für eine Gruppe der Formel

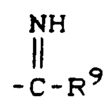

oder

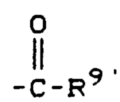

stehen,

worin

$R^9$ und $R^{9'}$ gleich oder verschieden sind und

- Wasserstoff, geradkettiges oder verzweigtes Alkyl mit bis zu 8 Kohlenstoffatomen oder Phenyl bedeuten,

$R^3$ und $R^5$ gleich oder verschieden sind und

- für geradkettiges oder verzweigtes Alkyl mit bis zu 8 Kohlenstoffatomen stehen, das gegebenenfalls durch Phenyl, Hydroxy, Halogen oder Amino substituiert ist,
- für Aryl mit 6 bis 10 Kohlenstoffatomen oder für einen 5- bis 7-gliedrigen, gesättigten oder ungesättigten Heterocyclus mit bis zu 4 Heteroatomen aus der Reihe N, O oder S stehen,

$R^4$

- für eine Gruppe der Formel $-NR^7R^8$, $-OR^{10}$ oder $-SR^{11}$ steht,

worin

$R^7$ und $R^8$ die oben angegebene Bedeutung haben,

$R^{10}$

- Wasserstoff oder geradkettiges oder verzweigtes Alkyl mit bis zu 8 Kohlenstoffatomen bedeutet, das gegebenenfalls durch Phenyl, Hydroxy, Halogen, geradkettiges oder verzweigtes Alkoxy mit bis zu 6 Kohlenstoffatomen oder durch einen gesättigten oder ungesättigten 5- bis 7-gliedrigen Heterocyclus mit bis zu 4 Heteroatomen aus der Reihe N, S oder O substituiert ist, oder
- Phenyl bedeutet, das bis zu 3-fach gleich oder verschieden durch Halogen, Trifluormethyl, Trifluormethoxy, Hydroxy oder durch geradkettiges oder verzweigtes Alkyl oder Alkoxy mit jeweils bis zu 6 Kohlenstoffatomen substituiert ist,

$R^{11}$

- Wasserstoff oder geradkettiges oder verzweigtes Alkyl mit bis zu 6 Kohlenstoffatomen bedeutet, das gegebenenfalls durch Hydroxy oder durch die Gruppe $-NR^7R^8$ substituiert ist,

worin

$R^7$ und $R^8$ die oben angegebene Bedeutung haben,

oder

- Phenyl oder einen 5- bis 7-gliedrigen, gesättigten oder ungesättigten Heterocyclus mit bis zu 4 Heteroatomen aus der Reihe N, S oder O bedeutet,

$R^6$

- für Wasserstoff, oder
- für geradkettiges oder verzweigtes Alkyl mit bis zu 8 Kohlenstoffatomen steht, oder
- für einen in vivo spaltbaren Esterrest steht,

und deren physiologisch unbedenklichen Salze.

**2.** 2-tert.substituierte Methyloxapenem-3-carbonsäuren der allgemeinen Formel (I)

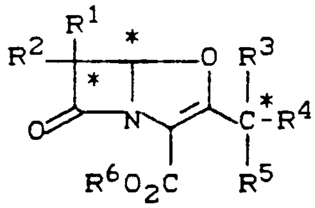

(I)

in welcher

$R^1$ und $R^2$ gleich oder verschieden sind und

- für Wasserstoff oder
- für geradkettiges oder verzweigtes Alkyl mit bis zu 6 Kohlenstoffatomen stehen, das gegebenenfalls durch Carboxy, Hydroxy, Phenyl, Phenoxy oder durch eine Gruppe der Formel $-NR^7R^8$ substituiert ist,

worin

$R^7$ und $R^8$ gleich oder verschieden sind und

- Wasserstoff, Phenyl oder geradkettiges oder verzweigtes Alkyl mit bis zu 6 Kohlenstoffatomen bedeuten,

oder

$R^7$ und $R^8$ gemeinsam mit dem Stickstoffatom einen Morpholino-, Tetrazolyl- oder Triazolylring bilden,

oder

$R^7$ oder $R^8$ für eine Gruppe der Formel

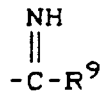

oder

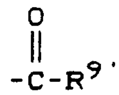

stehen

worin

$R^9$ und $R^{9'}$ gleich oder verschieden sind und

- Wasserstoff, geradkettiges oder verzweigtes Alkyl mit bis zu 6 Kohlenstoffatomen oder Phenyl bedeuten,

$R^3$ und $R^5$ gleich oder verschieden sind und

- für geradkettiges oder verzweigtes Alkyl mit bis zu 6 Kohlenstoffatomen stehen, das gegebenenfalls durch Phenyl, Hydroxy, Fluor oder Chlor substituiert ist,
- für Phenyl, Triazolyl, Pyridyl, Tetrazolyl, Thienyl, Oxazolyl, Isoxazolyl oder Thiazolyl stehen,

$R^4$

- für eine Gruppe der Formel $-NR^7R^8$, $-OR^{10}$ oder $-SR^{11}$ steht,

worin

$R^7$ und $R^8$ die oben angegebene Bedeutung haben,

$R^{10}$

- Wasserstoff oder geradkettiges oder verzweigte: Alkyl mit bis zu 6 Kohlenstoffatomen bedeutet, das gegebenenfalls durch Phenyl, Hydroxy, Tetrazolyl oder Triazolyl substituiert ist,
- Phenyl bedeutet, das gegebenenfalls bis zu 2-fach gleich oder verschieden durch Fluor, Chlor, Brom, Hydroxy oder durch geradkettiges oder verzweigtes Alkyl mit bis zu 4 Kohlenstoffatomen substituiert ist,

$R^{11}$

- Wasserstoff oder geradkettiges oder verzweigtes Alkyl mit bis zu 4 Kohlenstoffatomen bedeutet, das gegebenenfalls durch die Gruppe der Formel $-NR^7R^8$ substituiert ist,

  worin

  $R^7$ und $R^8$ die oben angegebene Bedeutung haben,
- Phenyl, Tetrazolyl, Triazolyl, Pyridyl, Thiazolyl, Oxazolyl oder Isoxazolyl bedeutet,

$R^6$

- für Wasserstoff, oder
- für geradkettiges oder verzweigtes Alkyl mit bis zu 6 Kohlenstoffatomen steht
- für einen Rest der Formel

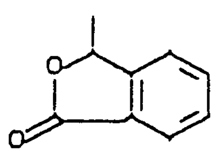 , 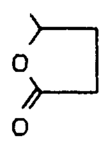 , 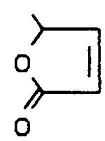 ,

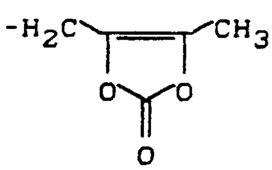 ,

$-CH_2-OCO-C(CH_3)_3$,

$-CH(CH_3)-OCOOC_2H_5$ oder

$-CH_2-OCOCH_3$ steht

und deren physiologisch unbedenklichen Salze.

**3.** 2-tert.substituierte Methyloxapenem-3-carbonsäuren der allgemeinen Formel (I)

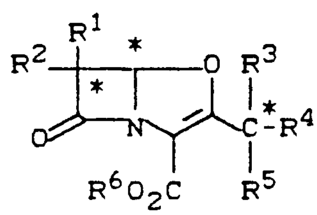

(I)

in welcher

$R^1$ und $R^2$ gleich oder verschieden sind und

- für Wasserstoff, oder
- für geradkettiges oder verzweigtes Alkyl mit bis zu 4 Kohlenstoffatomen stehen, das gegebenenfalls durch Carboxy, Hydroxy oder durch eine Gruppe $-NR^7R^8$ substituiert ist,

worin

$R^7$ und $R^8$ gleich oder verschieden sind und

- Wasserstoff oder geradkettiges oder verzweigtes Alkyl mit bis zu 4 Kohlenstoffatomen bedeuten,

$R^3$ und $R^5$ gleich oder verschieden sind und

- für geradkettiges oder verzweigtes Alkyl mit bis zu 4 Kohlenstoffatomen stehen, das gegebenenfalls durch Phenyl, Hydroxy oder Chlor substituiert ist,
- für Phenyl, Triazolyl, Tetrazolyl, Thienyl oder Thiazolyl stehen,

$R^4$

- für eine Gruppe der Formel $-NR^7R^8$, $-OR^{10}$ oder $-SR^{11}$ steht,

worin

$R^7$ und $R^8$ die oben angegebene Bedeutung haben,

$R^{10}$

- Wasserstoff oder geradkettiges oder verzweigtes Alkyl mit bis zu 4 Kohlenstoffatomen bedeutet, das gegebenenfalls durch Hydroxy substituiert ist,
- Phenyl bedeutet, das gegebenenfalls durch Fluor, Chlor oder Hydroxy substituiert ist,

$R^{11}$

- Methyl, Ethyl, Phenyl, Tetrazolyl, Triazolyl oder Thiazolyl bedeutet,

$R^6$

- für Wasserstoff oder
- für geradkettiges oder verzweigtes Alkyl mit bis zu 4 Kohlenstoffatomen steht

und deren physiologisch unbedenklichen Salze.

**4.** Verfahren zur Herstellung von Verbindungen gemäß Anspruch 1, dadurch gekennzeichnet, daß man Verbindungen der allgemeinen Formel (II)

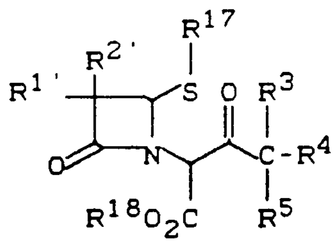

(II)

in welcher

$R^{1'}$ und $R^{2'}$ die oben angegebene Bedeutung von $R^1$ und $R^2$ haben oder für einen dort definierten Rest stehen, der durch eine Schutz- bzw. Markierungsgruppe substituiert ist,

$R^{17}$ - für geradkettiges oder verzweigtes Alkyl mit bis zu 6 Kohlenstoffatomen oder Phenyl steht,

und

$R^{18}$ - für eine Carboxyschutzgruppe steht,

zunächst in inerten Lösemitteln mit Halogenierungsmitteln zu Verbindungen der allgemeinen Formel (III)

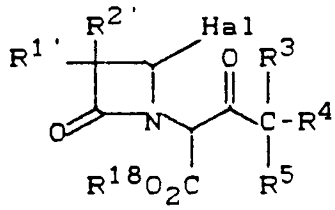

(III)

in welcher

$R^{1'}$, $R^{2'}$, $R^3$, $R^4$, $R^5$ und $R^{18}$ die oben angegebene Bedeutung haben,

und

Hal für Halogen, vorzugsweise für Chlor steht,

umsetzt, in einem nächsten Schritt unter Einwirkung von Basen in inerten Lösemitteln cyclisiert, und im Fall, daß $R^{1'}$ oder $R^{2'}$ für einen durch eine Schutz- oder Markierungsgruppe substituierten Rest steht, diese nach üblicher Methode, beispielsweise durch Hydrierung abspaltet.

**5.** Verbindungen der allgemeinen Formel (II)

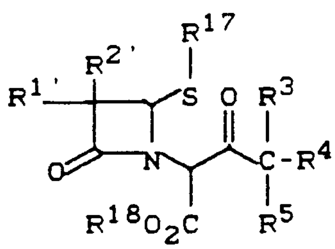

(II)

dadurch gekennzeichnet, daß

$R^1$ - $R^5$ die in Anspruch 1 angegebene Bedeutung haben,

$R^{17}$ für geradkettiges oder verzweigtes Alkyl mit bis zu 6 Kohlenstoffatomen oder Phenyl steht und

$R^{18}$ für eine Carboxyschutzgruppe steht.

**6.** Verbindungen der allgemeinen Formel (III)

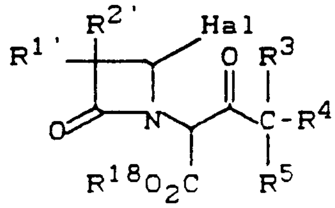

(III)

dadurch gekennzeichnet, daß

$R^1$ - $R^5$ die in Anspruch 1 angegebene Bedeutung haben,

Hal für Halogen und

$R^{18}$ für eine Carboxyschutzgruppe steht.

**7.** Pharmazeutische Zubereitung, die neben nicht-toxischen, inerten pharmazeutisch geeigneten Trägerstoffen mindestens eine Verbindung gemäß Ansprüchen 1 bis 3 enthält.

**8.** Pharmazeutische Zubereitung gemäß Anspruch 7, dadurch gekennzeichnet, daß sie neben den erfindungsgemäßen Verbindungen weitere pharmazeutische Wirkstoffe enthält.

**9.** Verwendung von 2-tert.substituierten Methyloxapenem-3-carbonsäuren der allgemeinen Formel (I) gemäß Anspruch 1 zur Herstellung von Arzneimitteln zur Behandlung von Krankheiten.

Europäisches Patentamt

# EUROPÄISCHER RECHERCHENBERICHT

Nummer der Anmeldung

EP 91114029.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | EP - A - 0 301 394 (BAYER AG) * Ansprüche * | 1,4, 6-9 | C 07 D 498/047<br>A 61 K 31/42<br>C 07 D 205/08 |
| D,A | EP - A - 0 362 622 (BAYER AG) * Ansprüche * | 7-9 | |
| A | GB - A - 1 591 438 (GLAXO LABORATORIES LIMITED) * Ansprüche 1,31 * | 1,4 | |
| A | DE - A - 3 010 531 (HOECHST UK LTD.) * Ansprüche 1,2 * | 4 | |
| D,A | DE - A - 2 517 316 (BEECHAM GROUP LTD.) * Ansprüche * | | |

RECHERCHIERTE SACHGEBIETE (Int Cl 5)

C 07 D 498/00
A 61 K

Der vorliegende Recherchenbericht wurde für alle Patentansprüche erstellt.

| Recherchenort | Abschlußdatum der Recherche | Prüfer |
|---|---|---|
| WIEN | 16-12-1991 | SCHNASS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument